# EUROPÄISCHE PATENTSCHRIFT

(11) **EP 3 669 778 B1**
(45) Veröffentlichungstag und Bekanntmachung des Hinweises auf die Patenterteilung: **07.07.2021**
(21) Anmeldenummer: 19210050.1
(22) Anmeldetag: 19.11.2019
(51) Int. Cl.: A61B 5/08, A61B 5/113, H04B 5/02, A61N 5/10, H01Q 21/06

(54) **DETEKTIONSVORRICHTUNG ZUM DETEKTIEREN EINER ATEMBEWEGUNG, COMPUTERPROGRAMM, COMPUTERLESBARES SPEICHERMEDIUM UND MEDIZINISCHES GERÄT**
DETECTION DEVICE FOR DETECTING A BREATHING MOTION, COMPUTER PROGRAM, COMPUTER READABLE STORAGE MEDIUM AND MEDICAL DEVICE
DISPOSITIF DE DÉTECTION POUR DÉTECTER UN MOUVEMENT RESPIRATOIRE, PROGRAMME D'ORDINATEUR, SUPPORT DE STOCKAGE LISIBLE PAR ORDINATEUR ET DISPOSITIF MÉDICAL

(30) Priorität: 17.12.2018 DE 102018221960
(43) Veröffentlichungstag der Anmeldung: 24.06.2020
(73) Patentinhaber: Siemens Healthcare GmbH, 91052 Erlangen (DE)
(72) Erfinder: MARTIUS, Sebastian, 91301 Forchheim (DE)

(56) Entgegenhaltungen:
- EP-A1- 2 117 077
- US-A1- 2015 002 331

## Beschreibung

Die Erfindung betrifft eine Detektionsvorrichtung und ein Verfahren zum Detektieren einer Atembewegung eines Patienten. Weiter betrifft die Erfindung ein entsprechendes Computerprogramm, ein entsprechendes computerlesbares Speichermedium und ein medizinisches Gerät mit einer entsprechenden Detektionsvorrichtung.

Viele gerätegestützte Verfahren in der Medizintechnik sind heutzutage auf genaue Positions- oder Bewegungsinformationen eines zu untersuchenden oder zu behandelnden Patienten angewiesen und/oder sind störanfällig gegenüber Positionsveränderungen oder Bewegungen des Patienten. Dies kann beispielsweise bildgebende Verfahren oder Anwendungen ebenso betreffen wie etwa eine onkologische Strahlentherapie. In letzter werden Tumore gezielt einer hohen Strahlenbelastung ausgesetzt, um sie dadurch in ihrem Wachstum zu hemmen oder idealerweise sogar abzutöten. Bei dieser Therapie soll möglichst wenig gesundes Gewebe in einer Umgebung des jeweiligen Tumors in Mitleidenschaft gezogen werden. Je nach Lage des jeweiligen Tumors kann sich dieser bedingt durch eine Atembewegung des jeweiligen Patienten während einer Behandlung bewegen, insbesondere aus einem Strahlengang oder Fokus einer zur Behandlung des Tumors vorgesehenen Strahlung. Somit kann dann umgebendes gesundes Gewebe statt des Tumors in den Strahlengang oder den Fokus geraten und in unerwünschter Weise geschädigt werden. Um dies zu vermeiden, ist also eine zielgenaue Bestrahlung notwendig, welche nur bei Kenntnis der Atembewegung erreichbar ist.

Bei bildgebenden Verfahren kann die Atembewegung hingegen beispielsweise zu verschwommenen oder verwischten Bildern führen, welche dann keine genaue oder zuverlässige Diagnose oder räumliche Zuordnung oder Registrierung mit anderen Daten erlauben.

Aus der heutigen Medizintechnik ist beispielsweise eine Verwendung von sogenannten Brust- oder Atemgürteln oder druck- oder dehnungssensorbasierten Atemdetektoren zum Detektieren der Atembewegung bekannt. Derartige Atemgürtel oder Atemdetektoren werden üblicherweise dem Patienten umgeschnallt und sind dadurch nicht für alle Anwendungen oder Einzelfälle geeignet, da sie beispielsweise in den Strahlengang geraten oder einen interventionellen Eingriff behindern können und zudem eine begrenzte Sensitivität aufweisen.

Die US 2015/002331 A1 offenbart ein Radarsystem mit einer Antennenanordnung, die flach ausgeführt ist, einschließlich einzeln betätigbarer Sendeeinheiten zum Senden von Radarsignalen und einzeln lesbarer Empfangseinheiten zum Empfangen von Radarsignalen. Die Sendeeinheiten und die Empfangseinheiten enthalten jeweils mindestens eine Antenne.

Die EP 2117077 A1 beschreibt eine Radarantennenanordnung, umfassend wenigstens eine erste Antennengruppe mit mehreren einzelnen, miteinander gekoppelten Antennenelementen, sowie wenigstens eine zweite Antennengruppe mit mehreren einzelnen, miteinander gekoppelten Antennenelementen, wobei die einzelnen Antennenelemente unterschiedlicher Antennengruppen galvanisch nicht miteinander verbunden sind, jedoch in einer Fläche derart verschränkt angeordnet sind, dass unmittelbar aufeinanderfolgende Antennenelemente unterschiedlicher Antennengruppen einander abwechseln.

Aufgabe der vorliegenden Erfindung ist es, eine verbesserte Detektion einer Atembewegung eines Patienten zu ermöglichen. Diese Aufgabe wird erfindungsgemäß durch die Gegenstände der unabhängigen Patentansprüche gelöst. Vorteilhafte Ausgestaltungen und Weiterbildungen der Erfindung sind in den abhängigen Patentansprüchen, in der Beschreibung und in den Figuren angegeben.

Eine erfindungsgemäße Detektionsvorrichtung dient, ist also eingerichtet zum Detektieren einer Atembewegung eines Patienten. Dazu weist die Detektionsvorrichtung wenigstens zwei metallische zumindest im Wesentlichen U-förmige Signalkopplungselemente auf. Diese Signalkopplungselemente sind dabei berührungsfrei ineinander verschachtelt, also ineinandergreifend, auf einem Substrat angeordnet, sodass jeweils zwischen zwei Seitensegmenten eines ersten der Signalkopplungselemente zumindest im Wesentlichen parallel dazu ein erstes Seitensegment eines zweiten der Signalkopplungselemente angeordnet ist. Ein zweites Seitensegment des zweiten Signalkopplungselements ist dabei außerhalb eines von dem ersten Signalkopplungselement dreiseitig umgriffenen Bereiches angeordnet.

Jedes der Signalkopplungselemente weist also zwei zumindest im Wesentlichen parallel zueinander angeordnete oder verlaufende, sich also erstreckende Seitensegmente und ein Bodensegment auf, welches jeweils die beiden Seitensegmente miteinander verbindet. Die Segmente können dabei beispielsweise lediglich durch ihre Anordnung oder Ausrichtung unterschieden sein, aber ebenso beispielsweise unterschiedliche Breiten und/oder Längen aufweisen. Die einzelnen Signalkopplungselemente können jeweils einstückig ausgebildet sein, sodass die einzelnen Segmente also nicht als getrennte Bauteile vorliegen müssen.

Weiter weist die erfindungsgemäße Detektionsvorrichtung eine mit den Signalkopplungselementen verbundene Auswerteelektronik auf. Diese Auswerteelektronik ist ausgebildet und eingerichtet zum Detektieren einer die Atembewegung indizierenden, also anzeigenden oder angebenden, oder charakterisierenden, Veränderung eines Empfangssignals. Dieses Empfangssignal wird dabei durch Einkopplung eines auf ein als Sender fungierendes der Signalkopplungselemente gegebenen Signals in ein zweites der Signalkopplungselemente in diesem erzeugt. Das zweite der Signalkopplungselemente fungiert dabei als Empfänger und ist in einem Nahfeldbereich des ersten Signalkopplungselements angeordnet. Letzteres kann insbesondere bedeuten, dass an einer Koppelstelle, an welcher das Signal von dem ersten Signalkopplungselement an das zweite Signalkopplungselement übertragen oder überkoppelt wird, ein Abstand zwischen den beiden Signalkopplungselementen geringer ist als 4λ oder als λ/2π, wobei λ eine vorgegebene Wellenlänge des für das Detektieren der Atembewegung auf das jeweils als Sender fungierendes der Signalkopplungselemente gegebenen Signals ist.

Die Wellenlänge λ kann beispielsweise durch rechtliche Vorgaben zur Vermeidung von Interferenzen oder durch für sprechende Anwendungszwecke gesetzlich vorgegebene oder freigehaltene Frequenzbänder vorgegeben sein. Insbesondere ist eine Größe oder Länge der Signalkopplungselemente auf die Wellenlänge λ abgestimmt, wobei die Signalkopplungselemente entlang ihres U-förmigen Verlaufs oder ihrer U-förmigen Erstreckung, also dem einen Seitensegment, dann dem Bodensegment und dann dem zweiten Seitensegment folgend, eine Länge von A/2 aufweisen können.

Bei der hier vorgeschlagenen Anordnung der Signalkopplungselemente, welche beispielsweise als Antennen aufgefasst werden können, ergibt sich zwischen jeweils zwei benachbart zueinander angeordneten Seitensegmenten unterschiedlicher Signalkopplungselemente wenigstens eine Koppelstelle, über welche hinweg das Signal von einem der Signalkopplungselemente in das nächste oder andere der Signalkopplungselemente gekoppelt wird. Im Bereich der Kopplung stellen verlaufen die Seitensegmente der jeweils zwei Signalkopplungselemente also zumindest abschnittweise zumindest im Wesentlichen parallel zueinander, sodass das Einkoppeln oder Überkoppeln oder Übertragen des Signals zwischen den beiden Seitensegmenten, also zwischen den beiden Signalkopplungselementen, als Übersprechen verstanden werden kann.

Ein Einkopplungs- oder Übertragungsverhalten kann dabei unter anderem von einem Abstand der beiden Signalkopplungselemente, insbesondere der beiden die jeweilige Koppelstelle bildenden oder einschließenden Seitensegmente, abhängen. Da die beiden Seitensegmente im Bereich der Koppelstelle zumindest im Wesentlichen parallel zueinander verlaufen, ergibt sich hier eine jeweilige Kapazität. Diese Kapazität kann durch äußere Einflüsse, beispielsweise in einen Bereich der Koppelstelle gebrachte Materialien oder Gegenstände oder Felder oder entsprechende Veränderungen oder Bewegungen, beeinflusst oder verändert werden. Eine solche Beeinflussung der Kapazität bewirkt wiederum eine Veränderung des Einkopplungs- oder Übertragungsverhaltens an der jeweiligen Koppelstelle. Wird also die Detektionsvorrichtung im Bereich des Patienten, beispielsweise unterhalb oder oberhalb des Patienten, angeordnet, so wird dessen Atembewegung zu einer Veränderung der Kapazität beziehungsweise des Einkopplungs- oder Übertragungsverhaltens an der jeweiligen Koppelstelle und somit - auch ohne Veränderung des auf das als Sender fungierenden Signalkopplungselements gegebenen Signals - zu einer Veränderung des resultierenden Empfangssignals führen.

Um die Atembewegung zu detektieren, also eine durch die Atembewegung bewirkte Veränderung des Empfangssignals beispielsweise von durch andere Effekte oder Einflüsse ausgelösten Veränderungen zu unterscheiden, kann beispielsweise eine entsprechende Kalibrierung durchgeführt werden. Ebenso kann beispielsweise als eine Bedingung vorgegeben sein, dass nur solche Veränderungen als Atembewegung oder als als Auswirkung der Atembewegung detektiert werden, welche sich wiederholt umkehren, also zumindest im Wesentlichen oder ungefähr rhythmisch oder regelmäßig oder periodisch wiederholen. Da eine typische Atmung nicht im streng mathematischen Sinne regelmäßig oder periodisch ist, können hier beispielsweise entsprechende Abweichungen zugelassen werden, beispielsweise durch entsprechende vorgegebene Intervalle, Schwellwerte, Wertebereiche oder dergleichen mehr. Als Atembewegung kann beispielsweise also eine fast-, pseudo- oder quasi-periodische Veränderung des Empfangssignals detektiert werden.

Ein besonderer Vorteil der vorliegenden Erfindung ist es, dass durch die hier vorgeschlagene Anordnung ein besonders kompakter Aufbau der Detektionsvorrichtung ermöglicht wird. Insbesondere können durch die verschachtelte Anordnung eine Vielzahl von Koppelstellen mit besonders hoher räumlicher Dichte realisiert werden, insbesondere im Vergleich zu anderen Antennentypen, wie beispielsweise einer einfachen rechteckigen Patchantenne. Es können also mehrere Signalkopplungselemente entsprechend verschachtelt, also als Kette oder Reihe, angeordnet werden. Dadurch wird vorteilhaft eine räumliche Auflösung bei der Detektion der Atembewegung ermöglicht. Durch die mittels der vorliegenden Erfindung für eine gegebene Wellenlänge oder Frequenz des zur Detektion verwendeten Signals erreichbare, vorteilhaft besonders hohe räumliche Dichte von Koppelstellen lässt sich dabei vorteilhaft eine besonders feine Abrasterung oder Abtastung eines durch die Detektionsvorrichtung abgedeckten Flächenbereiches, also eine besonders hohe Ortsauflösung der detektierten Atembewegung erreichen.

Ein weiterer Vorteil der vorliegenden Erfindung ist es, dass die Detektionsvorrichtung, beispielsweise im Vergleich zu herkömmlichen Atemgürteln, welche um eine Brust des Patienten gelegt werden, besonders flach und bauraumsparend gebaut werden kann. Beispielsweise kann die Anordnung aus den Signalkopplungselementen in eine Patientenliege oder einen Patiententisch integriert werden, auf den sich der jeweilige Patient dann legt. Somit kann die Atembewegung dann also ohne externe Sensoren und damit ohne Beeinflussung oder Behinderung einer Untersuchung oder Behandlung des Patienten, detektiert werden. Aufgrund der in den letzten Jahren und Jahrzehnten gemachten Fortschritte im Bereich der elektronischen Signalerzeugung, Signaldetektion und Signalverarbeitung, insbesondere einer erzielbaren Genauigkeit sowie Zeit- und Frequenzauflösung, kann die vorliegende Erfindung, welche auf einem elektronischen Messprinzip basiert, vorteilhaft die Atembewegung mit signifikant verbesserter Genauigkeit und Empfindlichkeit detektieren, insbesondere im Vergleich zu den angesprochenen Atemgürteln, welche beispielsweise auf einem zumindest teilweise mechanischen Prinzip basieren können.

In vorteilhafter Ausgestaltung der vorliegenden Erfindung weisen die Signalkopplungselemente jeweils ihrem U-förmigen Verlauf oder ihrer U-förmigen Erstreckung folgend gemessen eine Länge zwischen 16,1 cm und 17,6 cm, bevorzugt von 16,82 cm, auf. Diese Zahlenwerte können dabei eine Toleranz von beispielsweise 5% aufweisen. Diese Längen entsprechen also der Länge von einem Ende zum in Längserstreckungsrichtung gegenüberliegenden Ende des jeweiligen Signalkopplungselements wenn dieses aufgebogen würde, sodass es anstatt seiner U-förmigen Gestalt eine geradlinige Gestalt hätte. Da die Signalkopplungselemente wie beschrieben als Antennen fungieren oder aufgefasst werden können, wobei ihre Länge der halben Wellenlänge eines bevorzugt gesendeten oder empfangenen Signals entspricht, korrespondieren die hier vorgeschlagenen Längen der Signalkopplungselemente mit Signalfrequenzen von ungefähr 930 MHz bis ungefähr 853 MHz, bevorzugt ungefähr 892 MHz. Dies kann eine Verwendung der Signalkopplungselemente für unterschiedliche geeignete Frequenzbänder, insbesondere sowohl für Frequenzen von oder um 915 MHz als auch für Frequenzen von oder um 868 MHz, ermöglichen. Dies bedeutet wiederum, dass die erfindungsgemäße Detektionseinrichtung vorteilhaft besonders flexibel weltweit eingesetzt werden kann.

In weiterer vorteilhafter Ausgestaltung der vorliegenden Erfindung sind die Signalkopplungselemente jeweils aus einer metallischen Schicht mit einer Dicke von höchstens 50 µm, bevorzugt von weniger als 25 µm, gebildet. Die Dicke in diesem Sinne meint dabei eine Materialstärke der eigentlichen metallischen Signalkopplungselemente senkrecht zu ihrer jeweiligen Haupterstreckungsebene und damit entsprechend auch senkrecht zu einer Haupterstreckungsebene oder Haupterstreckungsrichtung des Substrats. Für einen praktischen Anwendungsfall können die Signalkopplungselemente beispielsweise aus einer ungefähr 17 µm dicken Schicht aus Aluminium, Kupfer oder einer entsprechenden Legierung oder einem ähnlichen metallischen Werkstoff gefertigt sein. Dies ermöglicht nicht nur einen vorteilhaft besonders dünnen und damit besonders einfach an jeweilige individuelle Bauraumgegebenheiten anpassbaren Aufbau der Detektionsvorrichtung, sondern kann die Signalkopplungselemente auch zumindest im Wesentlichen transparent für Röntgenstrahlung machen. Dadurch kann die erfindungsgemäße Detektionsvorrichtung vorteilhaft in röntgenbasierten medizinischen Geräten verwendet werden, ohne beispielsweise eine Strahlintensität oder eine resultierende Bildqualität zu beeinträchtigen. Besonders bevorzugt ist das Substrat so ausgebildet, dass eine Gesamtdicke oder Gesamthöhe eines Antennensystems der Detektionsvorrichtung, also des Substrats, der Signalkopplungselemente und gegebenenfalls vorhandener weiterer funktionaler Schichten oder Beschichtungen des Substrats, höchstens 0,5 mm beträgt. Damit kann die Detektionsvorrichtung beziehungsweise das Antennensystem vorteilhaft besonders einfach beispielsweise in einen Patiententisch eines medizinischen Geräts integriert werden und ist damit jederzeit verfügbar, konsistent betreibbar oder nutzbar und vor Beschädigungen geschützt.

In weiterer vorteilhafter Ausgestaltung der vorliegenden Erfindung ist das Substrat aus einem Kunststoffmaterial gebildet und auf einer von den Signalkopplungselementen abgewandten Rückseite metallisiert. Mit anderen Worten können die Signalkopplungselemente also auf einer der Rückseite gegenüberliegenden Oberseite des Substrats angeordnet oder aufgebracht sein. Diese Oberseite kann dann dem Patienten zugewandt angeordnet werden. Die metallisierte, also metallisch beschichtete, Rückseite des Substrats, welche von dem Patienten abgewandt angeordnet werden kann, kann dabei eine unerwünschte Beeinflussung der elektrischen Eigenschaften der Signalkopplungselemente, der Koppelstellen oder eines entsprechenden umgebenden Raum- oder Flächenbereiches verhindern oder verringern. Beispielsweise sind heutzutage verbreitete Patiententisch oder Patientenliegen medizinischer Geräte oftmals aus oder unter Verwendung von Kohlenstofffasern gefertigt, welche ohne die rückseitige durchgehende, ein bevorzugt vollflächige, Metallisierung des Substrats beispielsweise Sende- und/oder Empfangseigenschaften der Signalkopplungselemente verändern können und damit zu einer Beeinträchtigung der Funktion der Detektionsvorrichtung führen könnten. Ein solcher Einfluss wird vorliegend erfindungsgemäß durch die rückseitige Metallisierung jedoch verhindert oder soweit minimiert oder begrenzt, dass die Funktion der erfindungsgemäßen Detektionsvorrichtung nicht oder nicht signifikant beeinträchtigt wird, wenn diese auf einem entsprechenden Patiententisch oder oberflächennah in einen solchen Patiententisch integriert angeordnet und betrieben wird. Die Fertigung des Substrats aus dem Kunststoffmaterial, beispielsweise aus glasfaserverstärktem Kunststoff, stellt dabei nicht nur eine Möglichkeit zur kostengünstigen Fertigung der Detektionsvorrichtung mit etablierten Fertigungsmethoden dar, sondern kann vorteilhaft auch für eine elektrische Isolierung zwischen dem Signalkopplungselementen und der rückseitigen Metallisierung sorgen.

Ein weiterer Aspekt der vorliegenden Erfindung ist ein Verfahren zum Detektieren der Atembewegung des Patienten mittels der erfindungsgemäßen Detektionsvorrichtung. Bei dem Verfahren wird mittels der Auswerteelektronik ein vorgegebenes, bevorzugt kontinuierliches, Sendesignal auf genau ein erstes der Signalkopplungselemente gegeben. Die Auswerteelektronik kann dazu beispielsweise einen entsprechenden Signalgenerator, einen Verstärker und/oder dergleichen mehr aufweisen. Weiter wird bei dem erfindungsgemäßen Verfahren mittels der Auswerteelektronik ein durch Einkoppeln des Sendesignals aus dem ersten Signalkopplungselement über eine Koppelstelle hinweg in ein verschachtelt mit dem ersten Signalkopplungselement in dessen Nahfeldbereich angeordnetes zweites der Signalkopplungselemente in diesem resultierendes oder induziertes Empfangssignal gemessen.

In einem weiteren Verfahrensschritt des erfindungsgemäßen Verfahrens wird dann bei im Bereich des Patienten angeordneter Detektionsvorrichtung die Atembewegung als, insbesondere sich wiederholt umkehrende, Veränderung des Empfangssignals detektiert, welche ohne korrespondierende Veränderung des Sendesignals auftritt. Dies bedeutet, dass aufgrund der Atembewegung das Empfangssignal sich korrespondierend zu einem Atemrhythmus des Patienten beispielsweise wiederholt abschwächen kann mit jeweils dazwischenliegenden Signalanstiegen. Entsprechend kehrt sich dabei also eine Richtung oder ein Vorzeichen der Veränderung des Empfangssignals wiederholt um. Dies ist insbesondere dadurch bedingt, dass das Einkoppeln oder Übertragen des Sendesignals von dem jeweils als Sender fungierenden Signalkopplungselement in das jeweils als Empfänger fungierende Signalkopplungselement an der jeweiligen Koppelstelle beeinflusst wird oder abhängig ist von einer Kapazität der Koppelstelle oder an der Koppelstelle. Diese Kapazität wiederum kann insbesondere durch in einer Umgebung oder einem Bereich der Koppelstelle befindliche Wassermoleküle beeinflusst werden. Bei der Atembewegung des Patienten bewegen sich wasserhaltige Gewebebereiche des Patienten relativ zu der Koppelstelle, also relativ zu den Signalkopplungselementen und der Detektionsvorrichtung. Dies führt zu der Veränderung der Kapazität und damit des Einkopplungs- oder Übertragungsverhaltens an der Koppelstelle und damit zu einer elektronisch detektierbaren Veränderung des Empfangssignals. Diese Veränderung des Empfangssignals ist dabei insbesondere nicht korreliert mit einer Veränderung des Sendesignals. Das Sendesignal kann beispielsweise konstant oder in vorgegebener Weise veränderlich oder moduliert sein. Eine solche zeitliche Veränderung oder Modulation des Sendesignals würde jedoch, insbesondere ohne weitere äußere Beeinflussungen, stets direkt und in vorhersagbarer Weise mit einer entsprechenden zeitlichen Veränderung oder Modulation des Empfangssignals korrespondieren oder korreliert sein und beeinträchtigt daher nicht eine zusätzliche durch die Atembewegung bewirkte Veränderung des Empfangssignals oder deren Detektion.

Besonders bevorzugt kann die Auswerteelektronik dazu eingerichtet sein, durch Auswerten der Veränderung des Empfangssignals eine jeweils aktuelle Atemphase, also eine Phase oder einen Abschnitt der Atembewegung oder eines Atemzyklus, zu bestimmen. Solche Atemphasen, die automatisch bestimmt oder detektiert werden können, sind beispielsweise ein Einatmen und ein Ausatmen. Beispielsweise je nach Empfindlichkeit der verwendeten Detektionsvorrichtung und/oder beispielsweise je nach Anordnung des Patienten relativ zu der Detektionsvorrichtung können darüber hinaus weitere Atemphasen automatisch bestimmt oder detektiert werden, wie beispielsweise ein jeweiliger Anfang oder erster Teilabschnitt und/oder ein jeweiliges Ende oder letzter Teilabschnitt des Einatmens und/oder des Ausatmens und/oder beispielsweise ein zeitlicher Mittelpunkt des jeweiligen Atemzyklus und/oder beispielsweise ein Umkehrpunkt, in welchem sich eine Bewegungsrichtung zumindest eines Teilbereiches des Patienten relativ zu der Detektionsvorrichtung, beispielsweise zwischen dem Einatmen und dem Ausatmen, umgekehrt.

Besonders bevorzugt kann in Abhängigkeit von der vorliegend erfindungsgemäß detektierten Atembewegung oder Atemphase beispielsweise ein entsprechendes medizinisches Gerät gesteuert werden. Handelt es sich bei dem medizinischen Gerät beispielsweise um ein Bestrahlungsgerät, so kann die Bestrahlung gepulst sein und mit der detektierten Atembewegung oder Atemphase automatisch synchronisiert werden, sodass die Strahlung stets in derselben Atemphase oder demselben Punkt des Atemzyklus, bevorzugt also bei jeweils identischer Lage oder Position des Patienten, erfolgt. Damit kann beispielsweise ein Tumorgewebe besonders zuverlässig und zielgenau getroffen werden. Entsprechend kann beispielsweise ein bildgebendes medizinisches Gerät in Abhängigkeit von der erfindungsgemäß detektierten Atembewegung oder Atemphase automatisch hierzu synchronisiert angesteuert werden, sodass es jeweils in derselben Atemphase oder zum selben Zeitpunkt innerhalb des Atemzyklus ein Einzelbild aufnimmt. Dies führt vorteilhaft zu einer reduzierten Bewegungsunschärfe und einer vereinfachten Überlagerbarkeit oder Registrierung der Einzelbilder miteinander und somit letztlich zu einer verbesserten Bildqualität.

In weiterer vorteilhafter Ausgestaltung der vorliegenden Erfindung wird eine erfindungsgemäße Detektionsvorrichtung mit mehr als zwei Signalkopplungselementen verwendet, bei der die beiden Seitensegmente wenigstens eines der Signalkopplungselemente jeweils genau ein Seitensegment von zwei anderen der Signalkopplungselemente seitlich einschließen. Mit anderen Worten sind also die mehreren Signalkopplungselemente berührungsfrei in einer Längsrichtung oder einem mäanderförmigen Muster verschachtelt oder verkettet angeordnet. Beispielsweise können in einer Längsrichtung, welche sich beispielsweise zumindest im Wesentlichen senkrecht zu einer Längserstreckung der Seitensegmente der Signalkopplungselemente erstrecken kann, acht Signalkopplungselemente angeordnet.

Durch eine derartige reihenartige Anordnung der mehreren Signalkopplungselemente kann ein entsprechend größerer Flächenbereich abgedeckt werden, in welchem dann Atembewegungen mittels der Detektionsvorrichtung detektiert werden können. Dabei können ebenso mehrere derartige Reihen von in Längsrichtung verschachtelt angeordneten Signalkopplungselementen vorgesehen sein, um auch in Querrichtung, also quer zu der Längsrichtung, einen entsprechend größeren Flächenbereich abdecken zu können und eine entsprechende Ortsauflösung bei dem Detektieren der Atembewegung zu erreichen.

Gemäß der vorliegenden Ausgestaltung der vorliegenden Erfindung ist es vorgesehen, dass gemäß einem vorgegebenen Rhythmus ein als der Sender fungierendes der Signalkopplungselemente nach einem vorgegebenen Schema gewechselt wird. Mit anderen Worten kann zu einem ersten Zeitpunkt also ein erstes der Signalkopplungselemente als Sender fungieren. Nachdem ein mittels dieses Signalkopplungselements gesendetes oder übertragenes Signal als entsprechendes Empfangssignal gemessen wurde, kann dann zu einem späteren zweiten Zeitpunkt ein anderes der Signalkopplungselemente als Sender fungieren oder geschaltet werden. Insbesondere kann jeweils in einem solchen Schritt aus Senden und Empfangen oder Messen dasjenige der Signalkopplungselemente als Sender verwendet werden, welches in einem jeweils vorangegangenen Schritt als Empfänger fungiert hat. Dies kann so lange fortgesetzt werden, bis ein im aktuellen Schritt als Empfänger fungierendes der Signalkopplungselemente ein Ende der Anordnung aus Signalkopplungselementen bildet. Anschließend kann die Anordnung aus Signalkopplungselementen erneut, beispielsweise in umgekehrter Richtung oder nach einem vorgegebenen Muster, durch entsprechendes Wechseln des jeweils als Sender fungierenden Signalkopplungselements durchlaufen werden. Damit kann vorteilhaft ein Raum- oder Flächenbereich abgescannt oder abgetastet werden, die Atembewegung also ortsaufgelöst detektiert werden. Zudem kann dieses Vorgehen die Detektion der Atembewegung weniger anfällig hinsichtlich einer relativen Anordnung des Patienten und der Detektionsvorrichtung machen und gegebenenfalls eine genauere oder zuverlässigere Bestimmung oder Identifizierung der Atemphase ermöglichen, da sich während der Atembewegung, insbesondere innerhalb eines Atemzyklus, insbesondere in unterschiedlichen Atemphasen, verschiedene Bereiche des Patienten unterschiedlich bewegen können. Somit kann die Atembewegung besonders genau und ortsaufgelöst detektiert werden, beispielsweise bei einer Asymmetrie des Patienten, bei einer asymmetrischen Atembewegung und/oder bei einer ungenauen oder unbekannten Anordnung oder Ausrichtung des Patienten relativ zu den Signalkopplungselementen.

Ein weiterer Aspekt der vorliegenden Erfindung ist ein Computerprogramm oder Computerprogrammprodukt, welches Befehle oder Steueranweisungen umfasst, die bei einer Ausführung des Computerprogramms durch eine Datenverarbeitungseinrichtung, insbesondere eines medizinischen Geräts, diese dazu veranlassen, zumindest eine Ausführungsform des erfindungsgemäßen Verfahrens, insbesondere automatisch oder teilautomatisch, auszuführen. Das erfindungsgemäße Verfahren kann mit anderen Worten also die Verfahrensschritte des erfindungsgemäßen Verfahrens kodieren oder repräsentieren.

Ein weiterer Aspekt der vorliegenden Erfindung ist ein computerlesbares Speichermedium, auf dem zumindest eine Ausführungsform des erfindungsgemäßen Computerprogramms oder Computerprogrammprodukts gespeichert ist.

Ein weiterer Aspekt der vorliegenden Erfindung ist ein medizinisches Gerät, welches zumindest eine Ausführungsform der erfindungsgemäßen Detektionsvorrichtung aufweist. Das erfindungsgemäße medizinische Gerät kann beispielsweise ein Bestrahlungsgerät oder ein bildgebendes Gerät sein. Das erfindungsgemäße medizinische Gerät ist insbesondere zum Ausführen oder Durchführen des erfindungsgemäßen Verfahrens ausgebildet und eingerichtet. Dazu kann das erfindungsgemäße medizinische bildgebende Gerät, beispielsweise als Teil der Auswerteelektronik, ein erfindungsgemäßes computerlesbares Speichermedium und eine damit zum Ausführen des darauf gespeicherten erfindungsgemäßen Computerprogramms verbundene Prozessoreinrichtung, beispielsweise einen Mikroprozessor, Mikrochip oder Mikrocontroller, aufweisen. Das erfindungsgemäße medizinische Gerät kann also insbesondere das im Zusammenhang mit dem erfindungsgemäßen Verfahren genannte medizinische Gerät sein. Dementsprechend kann das erfindungsgemäße medizinische Gerät einzelne, einige oder alle der im Zusammenhang mit dem erfindungsgemäßen Verfahren genannten Eigenschaften und/oder Bauteile aufweisen.

In weiterer vorteilhafter Ausgestaltung der vorliegenden Erfindung weist das medizinische Gerät einen Patiententisch oder eine Patientenliege mit einer Liegefläche für den Patienten auf, wobei die Signalkopplungselemente der Detektionsvorrichtung in den Patiententisch, bevorzugt in die Liegefläche, integriert sind. Um eine Beschädigung der Signalkopplungselemente zu vermeiden, können diese beispielsweise durch eine elektrisch nicht leitende, insbesondere elektrisch permeable, Schutzschicht abgedeckt sein, wobei diese Schutzschicht dann eine Oberfläche des Patiententisches, insbesondere der Liegefläche, bilden kann. Somit können die Signalkopplungselemente dann also besonders oberflächennah angeordnet werden und damit die Atembewegung besonders genau und besonders zuverlässig detektieren, dabei aber gleichzeitig nicht in direkten Kontakt mit dem Patienten gelangen, wodurch nicht nur wie beschrieben eine Beschädigung, sondern ebenso beispielsweise ein Kurzschluss der Signalkopplungselemente vermieden werden kann.

Die bisher und im Folgenden angegebenen Eigenschaften und Weiterbildungen der erfindungsgemäßen Detektionsvorrichtung, des erfindungsgemäßen Verfahrens, des erfindungsgemäßen Computerprogramms, des erfindungsgemäßen computerlesbaren Speichermediums und des erfindungsgemäßen medizinischen Geräts sind wechselseitig zwischen diesen Aspekten der vorliegenden Erfindung übertragbar. Es gehören also zu der Erfindung auch solche Weiterbildungen der erfindungsgemäßen Detektionsvorrichtung, des erfindungsgemäßen Verfahrens, des erfindungsgemäßen Computerprogramms, des erfindungsgemäßen computerlesbaren Speichermediums und des erfindungsgemäßen medizinischen Geräts, welche Ausgestaltungen aufweisen, die hier zur Vermeidung unnötiger Redundanz nicht explizit in der jeweiligen Kombination oder nicht für jeden der Aspekte der vorliegenden Erfindung separat beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: einen bespielhaften schematischen Ablaufplan für ein Verfahren zum Detektieren einer Atembewegung eines Patienten;
- FIG 2: eine schematische Darstellung eines medizinischen Geräts mit einer Detektionsvorrichtung zum Detektieren einer Atembewegung eines Patienten;
- FIG 3: eine schematische Draufsicht auf ein Antennensystem einer Detektionsvorrichtung zum Detektieren einer Atembewegung;
- FIG 4: eine Dämpfungskurve zur Illustration eines elektrischen Verhaltens des Antennensystems aus FIG 3 bei verschiedenen Frequenzen.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche, funktionsgleiche oder einander entsprechende Elemente jeweils mit den gleichen Bezugszeichen gekennzeichnet.

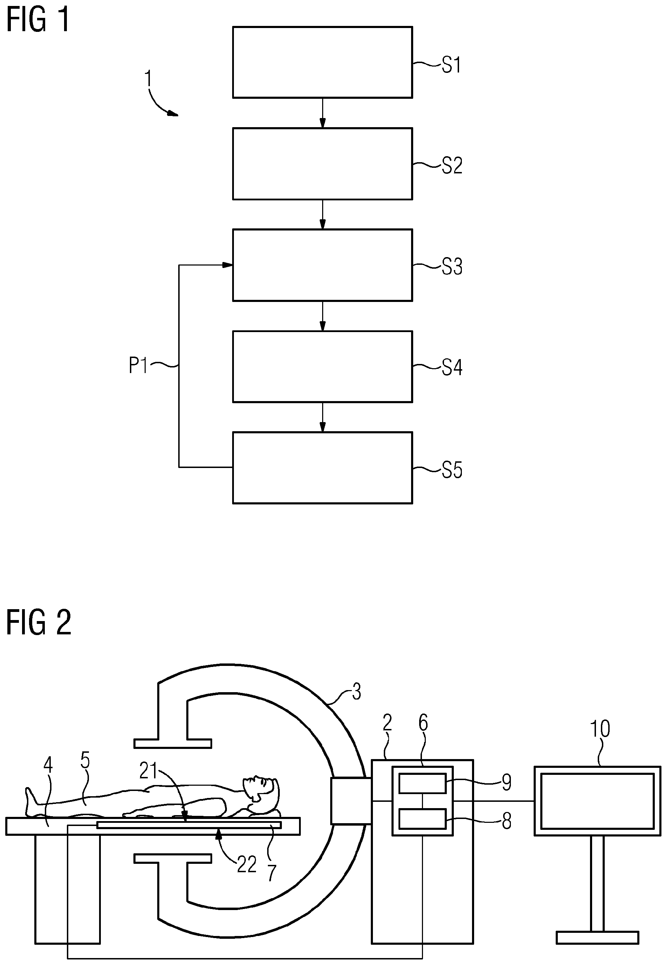

FIG 1 zeigt einen beispielhaften schematischen Ablaufplan 1 für ein Verfahren zum Detektieren einer Atembewegung eines Patienten 5 (siehe FIG 2). Dieses Verfahren kann beispielsweise mittels eines schematisch in FIG 2 dargestellten medizinischen Geräts 2 durchgeführt werden. Das medizinische Gerät 2 umfasst hier eine bewegliche, beispielsweise als C-Arm ausgebildete Halterung 3, an welcher beispielsweise eine Strahlenquelle für Bestrahlungszwecke oder zur Bildgebung angeordnet sein kann. Weiter umfasst das medizinische Gerät 2 einen Patiententisch 4, auf welchem vorliegend der Patient 5 in einem Einflussbereich des medizinischen Geräts 2, insbesondere beispielsweise von dessen Strahlungsquelle, angeordnet ist.

Weiter weist das medizinische Gerät 2 eine Datenverarbeitungseinrichtung 6 auf, welche mit einer in den Patiententisch 4 integrierten Antennenanordnung 7 gekoppelt ist. Die Datenverarbeitungseinrichtung und die Antennenanordnung 7 bilden vorliegend zusammen eine Detektionsvorrichtung zum Detektieren der Atembewegung des Patienten 5. Dazu kann die Datenverarbeitungseinrichtung 6 beispielsweise eine Signalerzeugungseinrichtung, also einen Signalgenerator, sowie eine Auswerteelektronik umfassen, um elektrische Signale auf die Antennenanordnung 7 geben und mittels der Antennenanordnung 7 gemessene oder empfangene Signale zu messen und auszuwerten.

Hierfür, also zur Durchführung des in FIG 1 schematisch illustrierten Verfahrens, weist die Datenverarbeitungseinrichtung 6 vorliegend ein computerlesbares Speichermedium 8 und eine damit verbundene Prozessoreinrichtung 9 auf. Auf dem Speichermedium 8 ist ein Computerprogramm gespeichert, welches die Verfahrensschritte des Verfahrens gemäß dem Ablaufplan 1 kodiert, also dessen Ausführung bewirkt, wenn es mittels der Prozessoreinrichtung 9 ausgeführt wird. Die Verfahrensschritte des Verfahrens, also des Ablaufplans 1, können also Programm- oder Funktionsmodule oder Codeblöcke des Computerprogramms sein oder repräsentieren.

Weiter ist hier eine Anzeigeeinrichtung 10 vorgesehen, welche mit der Datenverarbeitungseinrichtung 6 verbunden ist. Mittels der Anzeigeeinrichtung 10 kann beispielsweise die detektierte Atembewegung oder Atemphase, beispielsweise in Form einer Atemkurve und/oder als entsprechende Angabe der Atemphase, ausgegeben oder visualisiert werden. Die Datenverarbeitungseinrichtung 6 kann ebenso mit weiteren Einrichtungen des medizinischen Geräts 2 verbunden sein und beispielsweise als Steuergerät für diese Einrichtungen, beispielsweise die genannte Strahlenquelle, dienen, also eingerichtet sein.

Weltweit existieren unterschiedliche Frequenzbänder, in denen eine solche Detektionsvorrichtung regelkonform arbeiten, also betrieben werden kann oder darf. Vorliegend soll die Detektionsvorrichtung beziehungsweise die Antennenanordnung 7 möglichst alle entsprechenden Frequenzbänder abdecken. Weiter soll für eine möglichst einfache und flexible Positionierung eine Aufbauhöhe der Antennenanordnung 7 möglichst gering sein, beispielsweise höchstens 0,5 mm betragen. Weiter soll die Antennenanordnung 7 möglichst transparent für Röntgenstrahlen und gleichzeitig möglichst kompakt sein.

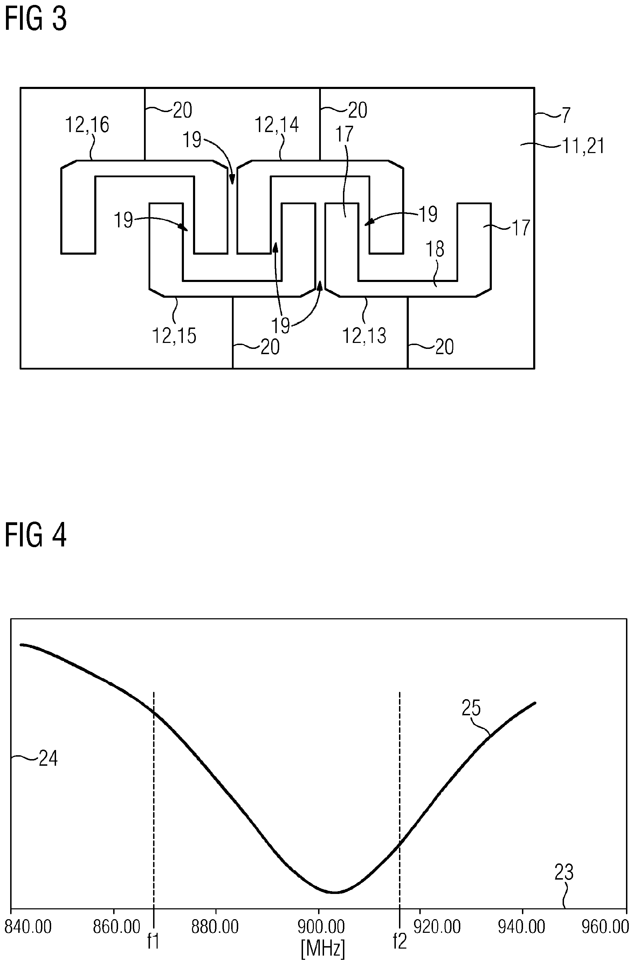

Um die Atembewegung des Patienten 5 detektieren zu können und gleichzeitig die genannten Anforderungen zu erfüllen, weist die Antennenanordnung 7 vorliegend wie schematisch in FIG 3 dargestellt eine verschachtelte Anordnung aus mehreren, auf einem aus Kunststoffmaterial gefertigten Substrat 11 angeordneten metallischen Signalkoppelelemente 12 auf. Vorliegend sind dies beispielhaft ein erstes Signalkoppelelement 13, ein zweites Signalkoppelelement 14, ein drittes Signalkoppelelement 15 und ein viertes Signalkoppelelement 16. Die Signalkoppelelemente 12 weisen hier jeweils zwei zumindest im Wesentlichen parallel zueinander angeordnete Seitensegmente 17 sowie ein Bodensegment 18, welches die beiden jeweiligen Seitensegmente 17 miteinander verbindet, auf. Die Seitensegmente 17 und Bodensegment 18 sind hier der Übersicht halber lediglich an dem ersten Signalkoppelelement 13 gekennzeichnet.

Durch die verschachtelte Anordnung der Signalkoppelelemente 12 erstrecken sich an mehreren Koppelstellen 19 jeweils zwei Seitensegmente 17 unterschiedlicher der Signalkoppelelemente 12 bereichsweise zumindest im Wesentlichen parallel zueinander. Über diese Koppelstellen 19 kann ein Sendesignal von einem als Sender fungierenden der Signalkoppelelemente 12 auf ein an der jeweiligen Koppelstelle 19 jeweils benachbartes der Signalkoppelelemente 12 übertragen werden, welches dann entsprechend als Empfänger fungiert. Wird beispielsweise das Sendesignal auf das erste Signalkoppelelement 13 gegeben, so kann durch Einkopplung des Sendesignals beispielsweise in das zweite Signalkoppelelement 14 an diesem ein entsprechendes Empfangssignal abgegriffen werden. Zusätzlich oder alternativ kann das Empfangssignal beispielsweise an dem dritten Signalkoppelelement 15 abgegriffen werden.

Zum Geben des Sendesignal auf das jeweils als Sender verwendete der Signalkoppelelemente 12 und zum Messen oder Abgreifen des entsprechenden Empfangssignals sind die Signalkoppelelemente 12 vorliegend durch jeweilige Anschlussleitungen 20 mit der Datenverarbeitungseinrichtung 6 elektrisch verbunden.

Bei dem in FIG 3 dargestellten Array aus den vier Signalkoppelelemente 12 können an den Koppelstellen 19 die jeweiligen Seitensegmente 17 beispielsweise ungefähr 7 mm voneinander beabstandet sein, wohingegen die einzelnen Koppelstellen 19 beispielsweise 30 mm voneinander beabstandet sein können. Damit ermöglicht die hier vorgeschlagene Ausgestaltung der Antennenanordnung 7 eine besonders kompakte Anordnung und eine besonders hohe räumliche Dichte der Koppelstellen 19, beispielsweise gegenüber einem Array aus für die gleiche Frequenz geeigneten Doppelpatchantennen, bei denen ein Abstand entsprechender Koppelstellen beispielsweise 100 mm betragen kann. Mit der vorliegenden Anordnung oder Antennenform der Antennenanordnung 7 beziehungsweise der Signalkoppelelemente 12 können also vorteilhaft dichter angeordnete Koppelstellen 19 realisiert werden als mit anderen Antennentypen. Dadurch ist vorteilhaft eine feinere Abrasterung des über der Antennenanordnung 7 liegenden Patienten 5 möglich.

Es ist hier Aufgabe der Antennenanordnung 7, möglichst effizient Leistung in Richtung des Patienten 5 abzustrahlen beziehungsweise von dort zu empfangen. Da der Patiententisch 4, in welchen die Antennenanordnung 7 integriert ist, vorliegend beispielsweise aus Carbon, also Kohlefaser, bestehen kann, könnte der Patiententisch 4 elektrische Eigenschaften, also eine Sende- und/oder Empfangseigenschaft oder -charakteristik der Antennenanordnung 7, insbesondere der Signalkoppelelemente 12, in unerwünschter Weise beeinflussen. Um diese Problematik zu umgehen oder zu minimieren, sind die Signalkoppelelemente 12 vorliegend auf einer dem Patienten 5 zugewandten Oberseite 21 des Substrats 11 angeordnet, wobei eine der Oberseite 21 gegenüberliegende Rückseite 22 des Substrats 11 durchgehend metallisiert ist. Die metallisierte Rückseite 22 dient dabei als elektrische oder elektromagnetische Abschirmung für die Signalkoppelelemente 12 gegenüber nicht durch die Atembewegung des Patienten 5 verursachten Einflüssen auf ein Signalübertragungsverhalten an den Koppelstellen 19.

Vorliegend weisen die einzelnen Signalkoppelelemente 12 ihrem jeweiligen U-förmigen Verlauf folgend gemessen eine Länge zwischen 16 und 18 cm vorliegend beispielsweise 16,82 cm auf. Damit sind sie sowohl für Signale von oder um 915 MHz als auch für Signale von oder um 868 MHz geeignet und somit weltweit flexibel einsetzbar. FIG 4 zeigt hierzu schematisch ein Diagramm, bei welchem auf einer x-Achse 23 eine Frequenz und auf einer y-Achse 24 eine Dämpfung aufgetragen ist. Eine in diesem Diagramm dargestellte Dämpfungskurve 25 für die Antennenanordnung 7 oder eine entsprechende konkrete Realisierung einer solchen Anordnung der Signalkoppelelemente 12 zeigt ein klares Minimum, vorliegend beispielhaft im Bereich zwischen 900 MHz und 908 MHz. Damit ist eine solche Antennenanordnung 7 aber dennoch mit den beiden international üblichen, hier gekennzeichneten Frequenzen f1 = 868 MHz und f2 = 915 MHz zum Detektieren der Atembewegung des Patienten 5 geeignet und betreibbar, da die Dämpfung bei den Frequenzen f1, f2 durch die entsprechende Abstimmung oder Anpassung der Signalkoppelelemente 12 und damit deren Dämpfungskurve 25 noch hinreichend gering ist. Bevorzugt kann also die Länge der Signalkoppelelemente 12 als A/2 ausgelegt werden für Signale mit einer Wellenlänge von λ_{Luft} = 336 mm, entsprechend einer Mittenfrequenz von 892 MHz, welche gerade mittig zwischen den Frequenzen f1 und f2 liegt.

In einem optionalen Verfahrensschritt S1 kann beispielsweise bei leerem Patiententisch 4, also ohne den Patienten 5 eine Übertragungscharakteristik der Antennenanordnung 7 über alle Koppelstellen 19 hinweg als Referenz gemessen werden. Dazu kann beispielsweise ein vorgegebenes Sendesignal der Reihe nach jeweils auf eines der Signalkoppelelemente 12 gegeben und ein resultierendes Empfangssignal als Referenzsignal gemessen werden.

Alternativ kann ein solches Referenzsignal bei bereits auf dem Patiententisch 4 angeordneten Patienten 5 gemessen werden, wobei der Patient beispielsweise seinen Atem in einer oder mehreren verschiedenen Atemphasen anhalten kann. Auf diese Weise kann eine Kalibrierung der Detektionsvorrichtung für den jeweiligen Patienten durchgeführt werden, wodurch dessen Atembewegung oder Atemphasen besonders genau und zuverlässig detektiert werden können.

In einem Verfahrensschritt S3 wird bei auf dem Patiententisch 4 angeordneten Patienten 5 zum Detektieren von dessen Atembewegung oder Atemphase das vorgegebene Sendesignal auf eines der Signalkoppelelemente 12, beispielsweise auf das erste Signalkoppelelement 13, gegeben. In einem Verfahrensschritt S4 wird dann an einem anderen der Signalkoppelelemente 12, beispielsweise an dem zweiten Signalkoppelelement 14, ein resultierendes Empfangssignal gemessen.

In einem Verfahrensschritt S5 wird dann das Empfangssignal ausgewertet, um die Atembewegung beziehungsweise die Atemphase zu detektieren oder zu bestimmen. Dabei kann insbesondere eine bei kontinuierlichem Sendesignal auftretende, sich wiederholt umkehrende Veränderung des Empfangssignals als Atembewegung detektiert werden. Das Empfangssignal oder dessen Veränderung oder Abweichung gegenüber dem Sendesignal kann also ausgewertet werden, um die Atembewegung oder Atemphase zu bestimmen. Dazu kann ebenso das Empfangssignal oder dessen Veränderung oder Abweichung gegenüber dem Sendesignal mit dem zuvor aufgenommenen Referenzsignal oder dessen Abweichung gegenüber dem Sendesignal verglichen werden.

Wie hier schematisch durch einen schleifenförmigen Programmpfad P1 angedeutet ist, kann das Sendesignal egelmäßig wiederholt oder kontinuierlich auf die Antennenanordnung 7 gegeben und die Atembewegung oder Atemphase durch entsprechende regelmäßig wiederholte oder kontinuierliche Auswertung überwacht werden. Ebenso kann bei jedem Durchlauf der Verfahrensschritte S3 bis S5 und des Programmpfads P1 das als Sender fungierende der Signalkoppelelemente 12 gewechselt werden. So kann beispielsweise in einem zweiten Durchlauf, beispielsweise nach einer vorgegebenen Zeitspanne, das Sendesignal auf das zweite Signalkoppelelement 14 gegeben werden, welches zuvor als Empfänger gedient hat. Dabei kann das Empfangssignal dann beispielsweise an dem dritten Signalkoppelelement 15 gemessen oder abgegriffen werden, und so weiter. Auf diese Weise können die Signalkoppelelemente 12 der Reihe nach durchlaufen werden, um einen gesamten durch die Antennenanordnung 7 abgedeckten Flächenbereich zum Detektieren der Atembewegung oder Atemphase abzutasten. Dadurch können Fehldetektionen der Atemphase besonders zuverlässig vermieden werden.

Insgesamt zeigen die beschriebenen Beispiele, wie eine U-förmige Patchantenne für eine Atemdetektion verwendet werden kann.

## Patentansprüche

1. Detektionsvorrichtung (6, 7) zum Detektieren einer Atembewegung eines Patienten (5), aufweisend
- wenigstens zwei metallische u-förmige Signalkopplungselemente (12), welche berührungsfrei ineinander verschachtelt auf einem Substrat (11) angeordnet sind, sodass jeweils zwischen zwei Seitensegmenten (17) eines ersten der Signalkopplungselemente (12; 13) parallel dazu ein erstes Seitensegment eines zweiten der Signalkopplungselemente (12; 14) angeordnet ist und ein zweites Seitensegment des zweiten Signalkopplungselements (12; 14) außerhalb eines von dem ersten Signalkopplungselement (12; 13) dreiseitig umgriffenen Bereiches angeordnet ist, und
- eine mit den Signalkopplungselementen (12) verbundene Auswerteelektronik (6, 9), welche eingerichtet ist zum Detektieren einer die Atembewegung indizierenden Veränderung eines durch Einkopplung eines auf ein als Sender fungierendes erstes der Signalkopplungselemente (12; 13) gegebenen Signals in ein als Empfänger in einem Nahfeldbereich des ersten Signalkopplungselements (12; 13) fungierendes zweites der Signalkopplungselemente (12; 14) in diesem erzeugten Empfangssignals,
**dadurch gekennzeichnet, dass** die Signalkopplungselemente (12) jeweils ihrem uförmigen Verlauf folgend gemessen eine Länge zwischen 16,1 cm und 17,6 cm, bevorzugt von 16,82 cm, aufweisen.

2. Detektionsvorrichtung (6, 7) nach Anspruch 1, **dadurch gekennzeichnet, dass** die Signalkopplungselemente (12) jeweils aus einer metallischen Schicht mit einer Dicke von höchstens 50 µm, bevorzugt weniger als 25 µm, gebildet sind.

3. Detektionsvorrichtung (6, 7) nach einem der vorhergehenden Ansprüche, **dadurch gekennzeichnet, dass** das Substrat (11) aus einem Kunststoffmaterial gebildet und auf einer von den Signalkopplungselementen abgewandten Rückseite (22) metallisiert ist.

4. Verfahren zum Detektieren einer Atembewegung eines Patienten (5) mittels einer Detektionsvorrichtung (6, 7) nach einem der vorhergehenden Ansprüche, wobei
- mittels der Auswerteelektronik (6, 9) ein vorgegebenes Sendesignal auf genau ein erstes der Signalkopplungselemente (12) gegeben wird,
- mittels der Auswerteelektronik (6, 9) ein durch Einkoppeln des Sendesignals aus dem ersten Signalkopplungselement (12; 13) über eine Koppelstelle hinweg in ein verschachtelt mit dem ersten Signalkopplungselement (12; 13) in dessen Nahfeldbereich angeordnetes zweites Signalkopplungselement (12; 14) in diesem resultierendes Empfangssignal gemessen wird,
- bei im Bereich des Patienten (5) angeordneter Detektionsvorrichtung (6, 7) die Atembewegung als, insbesondere sich wiederholt umkehrende, Veränderung des Empfangssignals detektiert wird, welche ohne korrespondierende Veränderung des Sendesignals auftritt.

5. Verfahren nach Anspruch 4, **dadurch gekennzeichnet, dass** eine Detektionsvorrichtung (6, 7) mit mehr als zwei Signalkopplungselementen (12) verwendet wird, bei der die beiden Seitensegmente (17) wenigstens eines der Signalkopplungselemente (12; 14, 15) genau jeweils ein Seitensegment von zwei anderen der Signalkopplungselemente (12; 13, 15; 14, 16) seitlich einschließen, wobei
- jeweils genau eines der Signalkopplungselemente (12) als Sender und ein damit verschaltetes der Signalkopplungselemente (12) als Empfänger fungieren, und
- gemäß einem vorgegebenen Rhythmus ein als der Sender fungierendes der Signalkopplungselemente (12) nach einem vorgegebenen Schema gewechselt wird.

6. Computerprogramm (1) umfassend Befehle, die bei einer Ausführung des Computerprogramms (1) durch eine Datenverarbeitungseinrichtung (6, 9), insbesondere eines medizinischen Geräts (2), diese dazu veranlassen, das Verfahren nach einem der Ansprüche 4 und 5 auszuführen.

7. Computerlesbares Speichermedium (8), auf dem das Computerprogramm (1) nach Anspruch 6 gespeichert ist.

8. Medizinisches Gerät (2), aufweisend eine Detektionsvorrichtung (6, 7) nach einem der Ansprüche 1 bis 3 und/oder eingerichtet zum Ausführen eines Verfahrens nach einem der Ansprüche 4 und 5.

9. Medizinisches Gerät (2) nach Anspruch 8, aufweisend einen Patiententisch (4) mit einer Liegefläche für den Patienten (5), wobei die Signalkopplungselemente (12) der Detektionsvorrichtung (6, 7) in den Patiententisch (4), bevorzugt in die Liegefläche, integriert sind.

## Claims

1. Detection apparatus (6, 7) for detecting a respiratory movement of a patient (5), comprising:
- at least two metallic u-shaped signal coupling elements (12), which are arranged on a substrate (11) such that they are interleaved without touching, so that between every two side segments (17) of a first signal coupling element of the signal coupling elements (12; 13) a first side segment of a second of the signal coupling elements (12; 14) is arranged parallel thereto, and a second side segment of said second signal coupling element (12; 14) is arranged outside a region surrounded on three sides by the first signal coupling element (12; 13); and
- analysis electronics (6, 9) connected to the signal coupling elements (12), which analysis electronics are configured to detect a change in a receive signal produced in a second of the signal coupling elements (12; 14) that is acting as a receiver in a near-field region of the first signal coupling element (12; 13) as a result of a signal given for a first of the signal coupling elements (12; 13) that is acting as a transmitter being coupled into said second of the signal coupling elements, which change indicates the respiratory movement,
**characterised in that** the signal coupling elements (12) each have a length of between 16.1 cm and 17.6 cm, preferably of 16.82 cm, measured along their U-shaped course.

2. Detection apparatus (6, 7) according to claim 1, **characterised in that** the signal coupling elements (12) are each made of a metallic layer of thickness 50 µm maximum, preferably of thickness less than 25 µm.

3. Detection apparatus (6, 7) according to one of the preceding claims, **characterised in that** the substrate (11) is made of a plastic material and metallised on a rear face (22) that faces away from the signal coupling elements.

4. Method for detecting a respiratory movement of a patient (5) by means of a detection apparatus (6, 7) according to one of the preceding claims, wherein
- the analysis electronics (6, 9) are used to place a defined transmit signal onto precisely one first signal coupling element of the signal coupling elements (12),
- the analysis electronics (6, 9) are used to measure a receive signal that, as a result of the transmit signal being coupled out of the first signal coupling element (12; 13) across a coupling point into a second signal coupling element (12; 14) that is interleaved with the first signal coupling element (12; 13) in its near-field region, arises in said second signal coupling element,
- with the detection apparatus (6, 7) arranged in the region of the patient (5), the respiratory movement is detected as a change, in particular a repeatedly reversing change, in the receive signal that occurs without a corresponding change in the transmit signal.

5. Method according to claim 4, **characterised in that** a detection apparatus (6, 7) having more than two signal coupling elements (12) is used, in which apparatus the two side segments (17) of at least one of the signal coupling elements (12; 14, 15) laterally enclose precisely one side segment each of two other signal coupling elements of the signal coupling elements (12; 13, 15; 14, 16), wherein
- precisely one of the signal coupling elements (12) in each case acts as a transmitter, and one of the signal coupling elements (12) that is connected thereto acts as a receiver, and
- one of the signal coupling elements (12) that is acting as a transmitter is interchanged on the basis of a defined scheme according to a specified rhythm.

6. Computer program (1) comprising commands, which on execution of the computer program (1) by a data processing facility (6, 9), in particular of a medical device (2), cause this facility to perform the method according to one of claims 4 and 5.

7. Computer-readable storage medium (8), on which is stored the computer program (1) according to claim 6.

8. Medical device (2) comprising a detection apparatus (6, 7) according to one of claims 1 to 3 and/or configured to perform a method according to one of claims 4 and 5.

9. Medical device (2) according to claim 8, comprising a patient table (4) having a reclining surface for the patient (5), wherein the signal coupling elements (12) of the detection apparatus (6, 7) are integrated in the patient table (4), preferably in the reclining surface.

## Revendications

1. Installation (6, 7) de détection pour la détection d'un mouvement respiratoire d'un patient, comportant
- au moins deux éléments (12) métalliques en forme de u de couplage de signal, qui sont disposés sur un substrat (11) en s'imbriquant l'un dans l'autre sans contact de manière à ce que respectivement entre deux segments (17) latéraux d'un premier des éléments (12; 13) de couplage de signal soit disposé parallèlement un premier segment latéral d'un deuxième des éléments (12, 14) de couplage de signal et de manière à ce qu'un deuxième segment latéral du deuxième élément (12; 14) de couplage de signal soit disposé à l'extérieur d'une région prise de trois côtés par le premier élément (12; 13) de couplage de signal, et
- une électronique (6, 9) d'exploitation, qui est reliée aux éléments (12) de couplage de signal et qui est conçue pour la détection d'une variation indiquant le mouvement respiratoire d'un signal de réception, produit dans un deuxième des éléments (12 ; 14) de couplage, par l'injection d'un signal donné sur un premier, servant d'émetteur, des éléments (12; 13) de couplage de signal dans le deuxième, servant de récepteur dans le domaine en champ proche du premier élément (12; 13) de couplage de signal, des éléments (12; 14) de couplage de signal, **caractérisée en ce que**
les éléments (12) de couplage de signal ont chacun, mesurés suivant leur tracé en forme de u, une longueur entre 16,1 cm et 17,6 cm, de préférence de 16,82 cm.

2. Installation (6, 7) de détection suivant la revendication 1, **caractérisée en ce que** les éléments (12) de couplage de signal sont formés chacun d'une couche métallique d'une épaisseur de 50 µm au plus, de préférence de moins de 25 µm.

3. Installation (6, 7) de détection suivant l'une des revendications précédentes, **caractérisée en ce que** le substrat (11) est en une matière plastique et est métallisée sur une face (22) arrière loin des éléments de couplage de signal.

4. Procédé de détection d'un mouvement respiratoire d'un patient (5) au moyen d'une installation (6, 7) de détection suivant l'une des revendications précédentes, dans lequel
- au moyen de l'électronique (6, 9) d'exploitation, on donne un signal d'émission donné à l'avance sur exactement un premier des éléments (12) de couplage de signal,
- au moyen de l'électronique (6, 9) d'exploitation, on mesure un signal de réception qui s'ensuit par injection du signal d'émission du premier élément (12; 13) de couplage de signal par l'intermédiaire d'un point de couplage dans un deuxième élément de couplage de signal imbriqué au premier élément (12; 13) de couplage de signal et disposé dans son domaine de champ proche,
- on détecte, alors que l'installation (6, 7) de détection est disposée dans la région du patient (5), le mouvement respiratoire sous la forme d'une variation du signal de réception, notamment s'inversant de manière répétée, signal de réception qui se produit sans variation correspondante du signal d'émission.

5. Procédé suivant la revendication 4, **caractérisé en ce qu'**on utilise une installation (6, 7) de détection ayant plus de deux éléments (12) de couplage de signal, dans laquelle les deux segments (17) latéraux d'au moins l'un des éléments (12; 14, 15) de couplage de signal enferment latéralement exactement respectivement un segment latéral de deux autres des éléments (12 ; 13, 15; 14, 16) de couplage de signal, dans lequel
- respectivement exactement l'un des éléments (12) de couplage de signal sert d'émetteur et l'un des éléments (12) de couplage de signal qui y est imbriqué de récepteur, et
- suivant un rythme donné à l'avance, on change suivant un schéma donné à l'avance l'un des éléments (12) de couplage de signal servant d'émetteur.

6. Programme (1) d'ordinateur comprenant des instructions qui, lorsqu'elles sont exécutées lors d'une exécution du programme (1) de l'ordinateur par un dispositif (6, 9) de traitement de données, notamment d'un appareil (2) médical, font que ce dispositif exécute le procédé suivant l'une des revendications 4 et 5.

7. Support (8) de mémoire déchiffrable par ordinateur, sur lequel le programme (1) d'ordinateur suivant la revendication 6 est mis en mémoire.

8. Appareil (2) médical comportant une installation (6, 7) de détection suivant l'une des revendications 1 à 3 et/ou conçu pour exécuter un procédé suivant l'une des revendications 4 et 5.

9. Appareil (2) médical suivant la revendication 8, comportant une table (4) de patient ayant une surface de couchette pour la patient (5), les éléments (12) de couplage de signal de l'installation (6, 7) de détection étant intégrés à la table (4) pour le patient, de préférence dans la surface de couchette.
